# EUROPEAN PATENT SPECIFICATION

(11) **EP 3 486 650 B1**
(45) Date of publication and mention of the grant of the patent: **12.03.2025**
(21) Application number: 18206546.6
(22) Date of filing: 15.11.2018
(51) Int. Cl.: G01N 33/18, C12Q 1/68, C02F 1/28, C02F 1/32, C02F 1/44, C02F 1/76, C02F 1/78, C02F 1/00, C02F 1/469, C02F 1/50, C02F 1/68, C02F 1/72, C12Q 1/70, C02F 3/00

(54) **METHOD FOR DETERMINING THE EFFECTIVENESS OF REMOVAL OF VIRUSES IN A PURIFICATION PROCESS**
VERFAHREN ZUR BESTIMMUNG DER WIRKSAMKEIT DER ENTFERNUNG VON VIREN IN EINEM REINIGUNGSVERFAHREN.
PROCÉDÉ PERMETTANT DE DÉTERMINER L'EFFICACITÉ DE L'ÉLIMINATION DE VIRUS DANS UN PROCÉDÉ DE PURIFICATION.

(30) Priority: 15.11.2017 NL 2019914
(43) Date of publication of application: 22.05.2019
(73) Proprietor: KWR Water B.V., 3433 PE Nieuwegein (NL)
(72) Inventor: HORNSTRA, Lucas Marinus, 3433 PE NIEUWEGEIN (NL); MEDEMA, Gerriet Jan, 3433 PE NIEUWEGEIN (NL); CORNELISSEN, Emile Robin, 3433 PE NIEUWEGEIN (NL); BEERENDONK, Eduard Franciscus, 3433 PE NIEUWEGEIN (NL)
(74) Representative: Algemeen Octrooi- en Merkenbureau B.V.

(56) References cited:
- ALBINANA-GIMENEZ N ET AL: "Analysis of adenoviruses and polyomaviruses quantified by qPCR as indicators of water quality in source and drinking-water treatment plants", WATER RESEARCH, ELSEVIER, AMSTERDAM, NL, vol. 43, no. 7, 4 February 2009 (2009-02-04), pages 2011 - 2019, XP026064721, ISSN: 0043-1354, [retrieved on 20090204], DOI: 10.1016/J.WATRES.2009.01.025
- XIAO YAN YE ET AL: "Real-Time PCR Detection of Enteric Viruses in Source Water and Treated Drinking Water in Wuhan, China", CURRENT MICROBIOLOGY, SPRINGER-VERLAG, NE, vol. 65, no. 3, 27 May 2012 (2012-05-27), pages 244 - 253, XP035088804, ISSN: 1432-0991, DOI: 10.1007/S00284-012-0152-1
- SIDHU JATINDER P ET AL: "Optimization of sampling strategy to determine pathogen removal efficacy of activated sludge treatment plant", ENVIRONMENTAL SCIENCE AND POLLUTION RESEARCH INTERNATIONAL, ECOMED, LANDSBERG, DE, vol. 24, no. 23, 28 June 2017 (2017-06-28), pages 19001 - 19010, XP036298478, ISSN: 0944-1344, [retrieved on 20170628], DOI: 10.1007/S11356-017-9557-5
- GWENALLE PIERRE ET AL: "Protocol for the assessment of viral retention capability of membranes", JOURNAL OF MEMBRANE SCIENCE, ELSEVIER BV, NL, vol. 381, no. 1, 6 July 2011 (2011-07-06), pages 41 - 49, XP028274005, ISSN: 0376-7388, [retrieved on 20110718], DOI: 10.1016/J.MEMSCI.2011.07.017
- BOFILL-MAS SILVIA ET AL: "Quantification and Stability of Human Adenoviruses and Polyomavirus JCPyV in Wastewater Matrices", vol. 72, no. 12, December 2006 (2006-12-01), US, pages 7894 - 7896, XP055875273, ISSN: 0099-2240, Retrieved from the Internet <URL:https://journals.asm.org/doi/pdf/10.1128/aem.00965-06> [retrieved on 20211221], DOI: 10.1128/AEM.00965-06

## Description

The present invention relates to a method for determining the effectiveness of removal of viruses in a purification process for producing an aqueous water stream for disinfection capacity and monitoring the integrity of purification processes.

In the production of drinking water from for example surface water but also from other sources, such as seawater, sewage treatment plant effluent, river-bank groundwater etc., it is necessary to remove viruses to a sufficient extent. This requires reliable methods of measurement. Water-transmitted viruses represent an important threat to drinking water safety. It is therefore important to know how many viruses are present in the surface water that is used for producing drinking water and whether these viruses are removed efficiently in various purification steps. Water companies collect data about the occurrence of viruses in surface water as a component of the legal Analysis of Microbiological Safety of Drinking Water (Analyse Microbiologische Veiligheid Drinkwater, AMVD) and for these companies it is therefore important to know how well their purification processes reduce the concentrations of viruses. In practice, for example samples were taken at an intake point of sewer water before and after purification, in particular coagulation/sedimentation and quick sand filtration, followed by determination of the extent to which various viruses were removed during this purification. The results obtained showed that during purification the quantity decreased by 0.7 log for unbound virus particles and 1.7 log for bound virus particles.

In an article by ALBINANA-EIMENEZ N. et al.: "Analysis of adenoviruses and polyomaviruses quantified by qPCR as indicators of water quality in source and drinking-water treatment plants", WATER RESEARCH, ELSEVIER, AMSTERDAM, NL, 43, No. 7, February 4, 2009 (2009-02-04), pages 2011-2019, XP026064-721, ISSN: 0043-1354, DOI: 10.1016/J.WATRES.2009.01.025 a method is described in which three drinking water treatment plants are investigated for the presence of human adenoviruses (HAdv) and JC polyomavirus (JCPyV), previously suggested as viral contamination indicators, in order to define their water quality in relation to the presence of viral pathogens and the efficiency of the treatments used. The concentration of said viruses in surface water is about 10³ (polyomavirus) and 10⁴ (adenovirus) gene copies per litre so that several concentration steps are necessary in order to reach an LRV (Log Removal Value) of approximately 4.

In the article by XIAO YAM YE et al.: "Real-Time PCR Detection of Enteric Viruses in Source Water and treated Drinking Water in Wuhan, China", CURRENT MICROBIOLOGY, SPRINGER-VERLAG, NE, 65, No. 3, May 27, 2012 (2012-05-27), 244-253, XP035088804, ISSN: 1432-0991, DOI: 10.1007/S00284-012-0152-1" a method is described in which water samples from water treatment plants are collected and analysed by real-time PCR assay for viral identification of enterovirus (EV), rotavirus group A (RVA), human adenovirus (HAdV) and human adenovirus subgroup F (HAdVF). The concentration operation mentioned in this article comprises membrane filtration, elution with beef extract and PEG precipitation. Concentration of this kind is complex and is not possible for routine analysis. Moreover, for the viruses investigated, the maximum LRV, if it is to be determined, is 1-2 (raw concentration:treated concentration: Enterovirus (10³-0:10³-0), Rotavirus A (10³-10⁴:10²-10⁴), Adenovirus (10³-10⁶:10²-10⁵) and Adenovirus F (10²-10⁴:10²-10³).

In the article by SIDHU JATINDER P et al.: "Optimization of sampling strategy to determine pathogen removal efficacy of activated sludge treatment plant", ENVIRONMENTAL SCIENCE AND POLLUTION RESEARCH INTERNATIONAL, ECOMED, LANDSBERG, DE, 24, No. 23, 28 June 2017 (2017-06-28), 19001-19010, XP036298478, ISSN: 0944-1344, DOI: 10.1007/S11356-017-9557-5, a method is described for describing the efficiency of removal of pathogens in wastewater treatment plants. The viruses to be detected here are enteroviruses (*E.coli*).

In the article by GWENALLE PIERRE et al.: "Protocol for the assessment of viral retention capability of membranes", JOURNAL OF MEMBRANE SCIENCE, ELSEVIER BV, NL, 381, No. 1, 6 July 2011 (2011-07-06), 41-49, XP028274085, ISSN: 0376-7388, DOI: 10.1016/J.MEMSCI, 2011-07-017, a method is described for determining the LRV of MS2 bacteriophage, suspended in various buffers, during filtration through hollow fibre membranes, manufactured from cellulose acetate. Thus, in this article, a marker, namely MS2, is added in order to determine membrane retention.

In an article written by Silvia Bofill-Mas et al "Quantification and Stability of Human Adenoviruses and Polyomavirus JCPyV in Wastewater Matrices", Applied and Environmental Microbiology, Dec. 2006, p. 7894-7896 the presence, quantity, and stability of HAdV and human polyomavirus JCPyV in different wastewater matrices is studied.

A method is known from American patent US 5,282,380 for nondestructive testing of an ultrafiltration membrane to determine the particle retention capacity of the membrane, wherein a first intrusion liquid and a wetting liquid, which is not miscible with the first intrusion liquid, are applied at a constant transmembrane pressure.

Various techniques are used for water purification, including membrane techniques, but also coagulation/sedimentation, rapid filtration, UV disinfection and passage through soil. For each of the purification techniques to be employed it is important to be able to establish the effectiveness of that purification technique. Especially in the removal of viruses it is necessary to find out how effectively they are removed in various purification steps.

Membrane filtration is being used increasingly for producing drinking water or clean process water. Especially in drinking water production it is very important to monitor membrane integrity. Particle breakthrough indicates a possible leak in a membrane and this requires immediate action. Not only is the end product of lower or poor quality, but there is also a high likelihood of biological regrowth. So-called membrane integrity monitors are also currently available commercially. Such a monitor is in fact an instrument that is based on particle counting, wherein even very low particle concentrations are detected directly. These low concentrations will not be detected by a turbidity meter.

Generally the term LRVs (log removal values) is employed in purification practice for quantifying the extent of removal. Such a value indicates to what extent removal takes place, namely 1-log decrease = 90%, 2-log decrease = 99%, 3-log decrease = 99.9%, 4-log decrease = 99.99% etc. If a minimum LRV ≥ 4 is desirable, only a certain number of techniques come into consideration for this, namely particles, nanoparticles, fluorescence techniques, Rhodamine-WT, TRASAR and MS2-phages. A disadvantage of these techniques is that in certain cases these employ dosage of substances in the source to be measured. The use of dosage of substances in feed water, for example colouring matter, (surrogate) phages and nanoparticles, is employed so as to be able to measure adequate removal of these substances. Dosage of substances in feed water for production of drinking water is seen as undesirable and entails additional costs.

The present inventors find that the aforementioned techniques are not sensitive enough, so that it is not possible to establish properly whether the purification processes adequately remove the viruses present in the water source. The maximum virus removal that can be established with the currently known techniques is about 4 log units. Concerning total purification, or other processes, in particular with dosage of phages, it is possible to measure and indicate even higher than 4.

The present inventors have in contrast the aim of obtaining, with a certain method, verification of the purification technique of for example 6 log units.

One aspect of the present invention is thus the provision of a method for determining the effectiveness of removal of viruses in a purification process for producing an aqueous stream.

Another aspect of the present invention is the provision of a method for determining the effectiveness of removal of viruses in a purification process for producing an aqueous stream wherein the addition of foreign substances becomes unnecessary.

Yet another aspect of the present invention is the provision of a method for determining the effectiveness of removal of viruses in a purification process for producing an aqueous stream wherein a log removal of more than 4 can be demonstrated.

Another aspect of the present invention is the provision of a method for determining the effectiveness of removal of viruses in a purification process for producing an aqueous stream wherein routine monitoring thereof is possible.

Another aspect of the present invention is the provision of a method for determining the effectiveness of removal of viruses in a purification process for producing an aqueous wherein laborious and complicated concentration steps become unnecessary.

The present invention thus relates to a method for determining the effectiveness of removal of viruses in a purification process for producing an aqueous stream, wherein the viruses present in water naturally are used for investigating the effectiveness of the purification process, said method comprising the following steps:
i) taking a water sample, at a point located upstream of the purification process;
ii) taking a water sample, at a point located downstream of the purification process;
   wherein the water sample is taken from a water source, selected from the group of salt water, brackish water, groundwater, surface water, seawater, sewage treatment plant effluent, storm water, rainwater, and process water, or a combination thereof;
iii) isolating genetic material from one or more viruses that are naturally present in the aforementioned water samples, obtained according to steps i) and ii), wherein the concentration of said one or more viruses in said water sample i) is in a range from more than one million to 10 billion per litre of water;
iv) carrying out a technique for multiplying RNA and/or DNA on the genetic material obtained according to step iii);
v) based on the results obtained from step iv), establishing the number of gene copies of specific virus types in the water sample obtained according to step i);
vi) based on the results obtained from step iv), establishing the number of gene copies of specific virus types in the water sample obtained according to step ii);
vii) determining the effectiveness of removal of viruses in the aforementioned purification process based on comparing the values obtained in step v) and vi),
wherein there is no dosage of markers to the water samples to be taken in step i) and/or step ii).

By applying the aforementioned steps i) - vii), one or more aspects of the present invention are satisfied. Thus, the present inventors have recognized that the viruses present in water naturally are used for investigating the effectiveness and integrity of purification processes. The aforementioned viruses are present in a very high concentration in the water to be purified, namely more than one million and up to possibly 10 billion per litre of water, and as a result according to the present method it has appeared possible to verify purification techniques with respect to removal of viruses with more than 6 log units. According to the present method, on the basis of a small piece of genome sequence of that virus, a molecular test is designed with which the virus may be indicated. By taking samples of water before and after the purification process, investigating this water for the numbers of the commonest viruses in the water, and reducing the numbers before the purification process by the numbers after the purification process, the removal of viruses may be quantified according to the present method. Because the viruses present in water naturally are used for investigating the effectiveness of the purification process, the present method does not employ dosage of one or more markers.

According to the present invention, the present method thus envisages taking a water sample before and after the purification process, after which the DNA and the RNA from the viruses that are present in the water samples are then isolated. Then, only in the case of a RNA virus, cDNA is synthesized from the RNA and a technique for multiplying RNA and/or DNA is carried out with primers on the DNA (DNA virus) or cDNA (RNA virus). Such a step is for example carried out with a set of natural viruses that is important for this purification process. The result of the multiplication technique is to be taken as the number of gene copies of this virus per litre, wherein the number of gene copies in the water after the purification process is reduced by the number of gene copies before the purification process, so that it is thus possible to determine how many gene copies, and thus how many viruses, are removed by the purification process that is employed. Thus, the present invention provides a method for evaluating the purification process.

According to the present invention, in step iii) the genetic material is DNA and/or RNA, or a combination thereof.

According to the present invention, if genetic material of the RNA type is present in step iii), for step iv) to be preceded by a step of synthesis of cDNA from the RNA.

As the technique employed in step iv) for multiplying RNA and/or DNA, one or more techniques are employed selected from the group of polymerase chain reaction (PCR), in particular of the qPCR type, nucleic acid sequence based amplification (NASBA), loop mediated isothermal amplification (LAMP), self-sustained sequence replication (3SR) and rolling circle amplification (RCA).

For a reliable and reproducible method it is desirable that the aforementioned one or more viruses from step iii) are determined beforehand. Determination of the aforementioned one or more viruses from step iii) beforehand takes place in particular on the basis of one or more aspects, selected from the group of concentration of the aforementioned one or more viruses in the water sample, size of the aforementioned one or more viruses, hydrophobicity of the aforementioned one or more viruses and isoelectric point of the aforementioned one or more viruses.

It is in particular desirable for the determination of the aforementioned one or more viruses from step iii) beforehand to take place on the basis of the number of viruses in the water sample. According to the present invention, the concentration of the aforementioned one or more viruses in the water sample is in a range from more than one million to 10 billion per litre of water.

The following may be mentioned as suitable purification processes that come into consideration for use in the present method: coagulation, flocculation, sedimentation, flotation, lamella separation, sand filtration, such as rapid filtration and (biological) slow sand filtration, membrane distillation, (biological) activated-carbon filtration, ion exchange, bank filtration and passage through soil, passage through sand dunes, pressure-driven membrane processes, such as microfiltration (MF), ultrafiltration (UF), nanofiltration (NF), reverse osmosis (RO), forward osmosis (FO), electrodialysis (ED), oxidative processes (which are also partly to be regarded as disinfection processes), such as AOP UV/H₂O₂/O₃, UV, Cl₂, ClO₂, O₃, UV/O₃, Fenton, and UV-peroxide treatment, ARP (Advanced reduction processes), in particular using UV and sulphite, adsorptive processes (AKF, IEX), biological processes (BAKF, BAZF, dune, bank, soil), dosing of water treatment chemicals, such as acids, antiscalants, biocides, coagulants (Fe, Al), flocculants, or a combination thereof.

It is in particular desirable that the aforementioned purification process comprises nanofiltration and/or reverse osmosis (RO).

Suitable water sources for taking a water sample as mentioned in steps i) and ii) are: salt water, brackish water, groundwater, surface water, seawater, sewage treatment plant effluent, storm water, rainwater, and process water, or a combination thereof. Semifinished products may also be mentioned, for example WRK water at Nieuwegein, or Dunea treatment at Brakel and Bergambacht.

For production of the aqueous water stream according to the present method, in particular the following types may be mentioned: irrigation water, process water, demineralized water, boiler feed water and drinking water, or a combination thereof.

The present method for determining the effectiveness of removal of viruses in a purification process for producing an aqueous stream is characterized in particular in that there is no dosing of markers in the water samples to be taken in step i) and/or step ii), said markers serving for determining the effectiveness of removal of viruses in a purification process for producing an aqueous stream.

According to an embodiment of the present method, the ratio of the volume of the water sample to be taken in step i) to the volume of the water sample to be taken in step ii) is in the range 1:1-1:10, preferably 1:1-1:5, wherein the magnitude of the volume of the water sample to be taken in step i) is in the range from 0.1 l to 1.5 l. Such a ratio seemed possible in particular because the present invention makes use of the viruses present in the water naturally, which are used for investigating the effectiveness of the purification process. Because the viruses being used in the present invention are present in large numbers in the water to be treated, the present inventors found that a high LRV can be established without complicated concentration steps. The present invention envisages in particular viruses that are present in a high concentration in the water sample to be investigated, wherein the presence of viruses in high concentration has the result that expensive, laborious and complicated concentration steps are not necessary. Routine monitoring is thus possible. Furthermore, the presence of viruses in high concentration makes it possible to obtain an LRV from >5 to even 7.

The present invention further relates to the use of measurement data obtained after carrying out a technique for multiplying RNA and/or DNA on genetic material, obtained after isolation thereof from one or more viruses that are present naturally in a water sample, for determining the effectiveness of removal of viruses in a purification process for producing an aqueous stream.

In a particular embodiment of the present invention the aim is to obtain an LRV (log removal value) of at least 4 log units, preferably at least 6, in particular at least 7.

The purification processes as mentioned in the particular use of the measurement data relate to the group of coagulation, flocculation, sedimentation, flotation, lamella separation, sand filtration, such as rapid filtration and (biological) slow sand filtration, membrane distillation, (biological) activated-carbon filtration, ion exchange, bank filtration and passage through soil, passage through sand dunes, pressure-driven membrane processes, such as microfiltration (MF), ultrafiltration (UF), nanofiltration (NF), reverse osmosis (RO), forward osmosis (FO), electrodialysis (ED), oxidative processes (which are also partly to be regarded as disinfection processes), such as AOP UV/H₂O₂/O₃, UV, Cl₂, ClO₂, O₃, UV/O₃, Fenton, and UV-peroxide treatment, ARP (Advanced reduction processes), in particular using UV and sulphite, adsorptive processes (AKF, IEX), biological processes (BAKF, BAZF, dune, bank, soil), dosing of water treatment chemicals, such as acids, antiscalants, biocides, coagulants (Fe, Al), flocculants, or a combination thereof, in particular nanofiltration and/or reverse osmosis (RO).

Examples of water sources, as mentioned in the particular use of the measurement data, are: salt water, brackish water, groundwater, surface water, seawater, sewage treatment plant effluent, storm water, rainwater, and process water, or a combination thereof. Semifinished products may also be mentioned, for example WRK water at Nieuwegein, or Dunea treatment at Brakel and Bergambacht.

The present inventors find that with the present method according to the invention, it may be established that an intact membrane achieves a very high level of virus removal, but that if the membrane is damaged, said removal can no longer be obtained. This can clearly be seen in Table A. Thus, the measured LRVs (log removal values) are many times higher for the intact membrane than for the damaged membrane. The present invention thus makes it possible to establish that viruses are removed efficiently in the membrane installation and that this removal decreases when the membrane appears to be damaged, said damage being deduced from the change in LRV.

**Table A: determination of the extent of virus removal in i) an intact membrane and ii) a damaged membrane.**

| i) Intact membrane | | | | |
|---|---|---|---|---|
| | gene copies/l primer A | gene copies/l primer B | Log gene copies/l primer A | Log gene copies/l primer B |
| Influent | 2.3E+07 | 4.2E+08 | 7.37 | 8.63 |
| Effluent | below detection limit | below detection limit | 0.00 | 0.00 |
| Virus removal by membrane: | | | > 7.37 Log | > 8.63 Log |

| ii) Intact membrane | | | | |
|---|---|---|---|---|
| | gene copies/l primer A | gene copies/l primer B | Log gene copies/l primer A | Log gene copies/l primer B |
| Influent | 2.3E+07 | 4.2E+08 | 7.37 | 8.63 |
| Effluent | below detection limit | below detection limit | 0.00 | 0.00 |
| Virus removal by membrane: | | | > 7.37 Log | > 8.63 Log |

The present invention will be explained in more detail hereunder based on an example, wherein it must be clear that the example is not to be seen as a limitation of the scope of protection but merely as an explanation of the present invention.

For concentrating the water sample, a protocol is applied, starting from an amount of 1 litre of the water to be sampled. To minimize the variation of the composition of the sample between the sampling time and analysis, it is desirable for the sample to be transported on ice and stored at a temperature between 0 and 8°C. Then the sample is filtered by means of a 0.4 µm polycarbonate filter under vacuum, after which the residue is collected in a bottle. The sample is then concentrated by means of Centricon Plus-70 (Merck UFC710008). The following steps are used for this: weighing the sample bottle with the sample to be analysed, pouring the sample into the Centricon beaker with the filters (filtration beaker), placing the filtration beaker in the filtration collection cup and closing the beaker at the top with a cover and setting up the centrifuge (Accel: 9, Decel: 9, Speed: 3000 x g, Time: 10 minutes, Temperature: 4°C). After centrifuging the sample, the filtrate is collected in the collecting beaker, after which the filtrate is removed from the collecting beaker and the sample is centrifuged again in the filtration beaker. The last two steps are repeated. In the last centrifugation, the filtrate is kept in the collecting beaker and placed in the centrifuge, after which the residue is in the receiving tank. The sample bottle is weighed and the difference between the empty bottle and the sample bottle with the analysis sample is the volume used in the calculations.

DNA isolation is carried out using the DNeasy^{®} PowerBiofilm^{®} Kit from the supplier Qiagen (Art. No. 24000-50). The protocol supplied by the manufacturer of this kit is followed.

For RNA isolation for RNA viruses, the PowerMicrobiome (trademark) RNA Isolation Kit is used, wherein the protocol supplied by the manufacturer of this kit is followed.

For cDNA synthesis, the iScript ^{®} cDNA Synthesis Kit, marketed by Bio-Rad, is used. The protocol supplied by the manufacturer of this kit is followed.

With the help of Next generation sequencing, the present inventors found genetic information of many viruses from raw water, from which a selection was then made. Next, comparison is made with a database, from which it was found that the virus sequences (DNA or RNA sequence) look just like the viruses in the following table. They may therefore be these viruses, or viruses related to them. From the practical viewpoint, the present inventors gave the viruses definite names, namely the four-digit numbers in the 1st column of Tables 1, 2, 3 and 4.

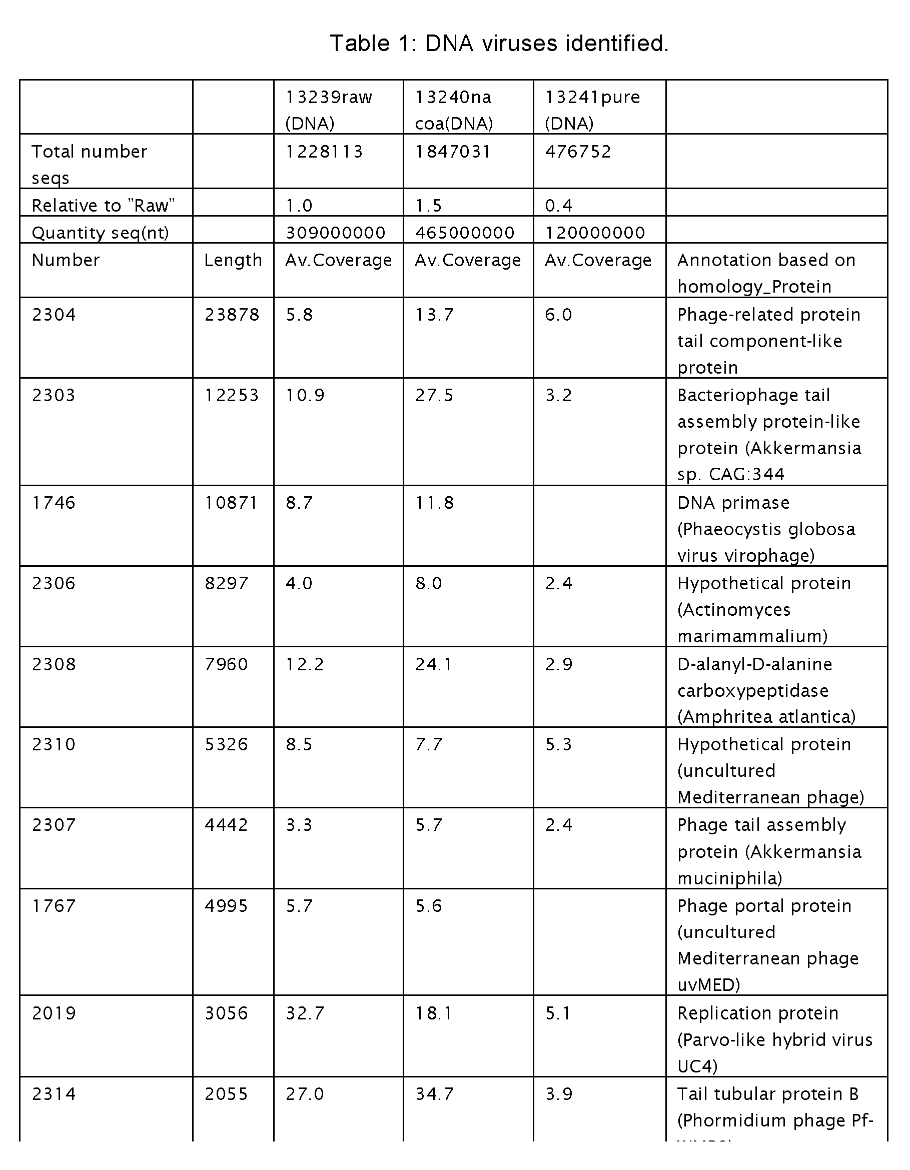

Based on the DNA or RNA sequence, the present inventors developed a PCR reaction with which the specific virus can be detected by means of Q-PCR, for which a primer pair 1 and a primer pair 2 are used, namely small pieces of DNA that are identical to the DNA sequence in a small piece of the DNA or RNA of the virus to be detected. It is then possible to detect a virus specifically in the water samples.

**Table 2: Primer sequences for identifying and quantifying the DNA viruses by Q-PCR.**

| DNA Viruses | | | | | | | |
|---|---|---|---|---|---|---|---|
| Contig | | Primer pair 1 | | | Primer pair 2 | | |
| Contig No. | Contig length | f | r | Fr length | f | r | Fr length |
| 2314 | 2055 | ACCAGGGGCGGTGTATATTG | GACGCCGTTGAAATGTCAGG | 102 | TGTCGGAGCGTTTCCAATGA | TTGTCAACCCACGTTGGAGC | 90 |
| 2303 | 12253 | GCCATAATTGGCTTCAGCGG | TCGCGCACTTGGTCAAAAAG | 100 | TTAAAGATGCCTTGCCCGGT | GCATTCGGGAAAGCGGAATC | 83 |
| 2304 | 23878 | TTACGCCAGCCAAGGTGAAT | ATCTTTCAAGCCGCCCATGA | 103 | TGGTAACCGTGAACATCCCG | ACTGCTGATCGCAATCCCAA | 98 |
| 2306 | 8297 | TTACGCCAGCCAAGGTGAAT | TCTAGTTGCTGCCGTTGGAG | 96 | TTGTTCGACTAATCCGGCCC | CCCATTGTCGTCAACGGAGA | 84 |
| 1746 | 10871 | ACCGAAACACCAAACCACCT | ACCGCATAGAATCCAGCGAG | 90 | CGAGAAGACACTCGGACTCG | CGCATCTGAAGCCGCTAAAC | 103 |
| 1767 | 4995 | TGTTCCGGTAGCTTTCTGCC | ATCGGGATTCAGTATGGGGC | 110 | TGCCCCATACTGAATCCCGA | TCGGAGCAACATCAGCAATAGA | 85 |
| 2310 | 5326 | GCATCTTCGTCAATGCGTCC | GAGGTCGTGGTGTGGCTATC | 91 | TTGAGAAGCGAGCCGAGAAG | GGATAGCGTCTCGTCGATGG | 92 |
| 2307 | 4442 | TGGACTCCAACGCTCACATC | CGTTATTTCAGCGTCCTGCG | 81 | CGCCATGTGGGCAAAAATGA | CGCTTAAATCCTGCGAACCG | 89 |
| 2308 | 7960 | GCGATGACCGCTCATTTGTC | CGCTGTCTGCTGTGAGTGTA | 105 | GGGCGAAACTACCAACCAGA | GTATGGTAGCGATGCCGTGA | 106 |
| 2019 | 3056 | ACCTCGTGGTAGAGACCCAA | CATCGGGGTGTCTGCTTGAT | 97 | GCCTTACACCGGTTGGAGAA | CGGTCAGCTGGTAAGGTTCC | 99 |

**Table 3 RNA viruses identified.**

| | **Seqs rel>** | 1.00 | 0.96 | 0.85 | |
|---|---|---|---|---|---|
| | **Number seqs>** | 626022 | 602682 | 534989 | |
| **Contig** | **Sample>** | 13239 **(Raw)** | 13240 **(Na coag)** | 13241 **(Pure)** | |
| **Number** | **Length** | **Av.Coverage** | **Av.coverage** | **Av.coverage** | **Homology_Protein** |
| 2591 | 11194 | *57.8* | *3.8* | *3.8* | RdRp [Wenling hepe-like virus 4] |
| 343 | 9345 | *14.6* | *14.8* | *3.0* | Sanxia picorna-like virus 7 strain |
| 2771 | 9334 | *11.0* | *10.4* | *7.5* | hypothetical protein [Hubei picorna-like virus 48] |
| 2682 | 9026 | *40.2* | *27.8* | *16.5* | hypothetical protein 1 [Beihai picorna-like virus 17] |
| 67 | 9010 | *83.4* | *119.0* | *36.7* | hypothetical protein 2 [Shahe picorna-like virus 11], family of No. 101? |
| 239 | 8644 | *232.2* | *183.1* | *196.9* | hypothetical protein [Changjiang picorna-like virus 2] |
| 355 | 4532 | *22.9* | *52.4* | *28.9* | ORF2b [Artemisia virus A] |
| 2700 | 2012 | *5.1* | *4.1* | *3.1* | maturation protein [Caulobacter phage phiCb5] |
| 1028 | 2016 | *11.0* | *8.7* | *11.1* | hypothetical protein 2 [Hubei leech virus 4] |
| 2885 | 2019 | *22.1* | *12.9* | *10.1* | replicase [Johnsongrass chlorotic stripe mosaic virus] |

**Table 4: Primer sequences for identifying and quantifying the RNA viruses by Q-PCR.**

| **RNA viruses** | | | | | | | |
|---|---|---|---|---|---|---|---|
| | **Contig** | **Primer pair 1** | | **Fragment** | **Primer pair 2** | | **Fragment** |
| **Contig No.** | **Length** | **F** | **R** | **Length** | **F** | **R** | **Length** |
| 2591 | 11194 | TGTTGAAAAAGGTGCTGCGG | GACTGCCCTTGCTCAGAAGT | 82 | AGCGTTGGAGAATTCGTGGT | GTGCTGCCTCCTAACGGAAT | 82 |
| 343 | 9345 | TTGTCCACCCGTGCTCATAC | CTACAGCGTCTCCTGCGAAT | 83 | CTCAAATCGGTTCTGGCCCT | CCGCAAGCCTTGATGGAAAG | 80 |
| 2771 | 9334 | ACTAGCCATTTCTGCCCACC | AGCAGTTTTGGACTCGCGTA | 103 | ATGCGTTGCCATTTTCCCAC | CCTGGCAAGATTAGCGTCCA | 82 |
| 2682 | 9026 | CCAAACAAGGTTGCTACCGC | TACGCTCGTATGCTCGTCAC | 98 | AGGGATCGTTCAGCCACAAG | ATGTGCAGTCGTGGATTCGT | 85 |
| 2700 | 2012 | GCAAAGGATAGCTCGCAACG | TGGCAGGAAGTGGTCGATTC | 87 | ATAGAATGTACTGGCCGCCG | GTGGAGCTCCGAGGTATTCG | 94 |
| 2556 | 8948 | TGTTGCGATCCTTCCAGTCC | AGCGATGATGTGGATGCCAA | 106 | ACGGGAACACTTGCATCCTT | GTTACACGCCGACACCAAAC | 84 |
| 67 | 9010 | GTACTGACGTGTCAGCTGGT | ACCTGCGTCTTGGGTTACTG | 80 | CTGCCGTCCAGTTTGGTTTG | GAGCGGACGCACAATTTGAT | 87 |
| 239 | 8644 | CTATCGCAAACCCGCAACAGCC | CAAACATGGTCGCGTGATGA | 110 | AAGTATGCCCCAGGTCGTTG | ACTGGCAGGTGCACATAGTA | 87 |
| 1028 | 2016 | TGCTCGGAGGTTGAGATGTG | TAAGGTGAGCGTTCGAGCAG | 89 | GAGCCCTTGGCAGACTTACA | CTGGTTCCGGATCGGACTTC | 81 |
| 355 | 4532 | TGAGTCATCCCCCATGGCTA | AGGCTCGT ACCT AACGTCCT | 97 | CGCACTAAGAAGGGTGCAGA | AGGGTCCATCTGTCCTCGAA | 90 |
| 2885 | 2019 | GACATGCGGCATCCATCAAC | CCGAAATGCTGACCATGCAG | 91 | GTCTGAAACGCGATTGGTGG | TGACCATTATCCCGCTCACG | 102 |

**Table 5: The Ct values of ten natural viruses in water sampled before and after a RO membrane.**

| | | | | | | | | | | |
|---|---|---|---|---|---|---|---|---|---|---|
| Primer set DNA | 1746 | 1767 | 2019 | 2303 | 2304 | 2306 | 2307 | 2308 | 2310 | 2314 |
| Kamerik Opp. Water | 25.1 | 25.7 | 35.1 | 26.2 | 30.1 | 25.6 | 25.7 | 23.0 | 19.4 | 19.9 |
| Kamerik after RO | N/A | N/A | 39.3 | N/A | N/A | N/A | N/A | N/A | N/A | N/A |
| DCq after RO Opp water | > 14.9 | > 14.3 | > 4.9 | > 13.8 | > 9.9 | > 14.4 | > 14.3 | > 17.0 | > 20.6 | > 20.2 |
| Log difference | > 4.5 | > 4.3 | 1.5 | > 4.2 | > 3.0 | > 4.4 | > 4.3 | > 5.1 | > 6.2 | > 6.1 |
| Kameri k Groundwater | N/A | N/A | N/A | N/A | N/A | N/A | N/A | N/A | 39.8 | N/A |
| Water Lekkanaal (WRK) | 29.4 | 23.1 | 30.9 | 27.0 | 24.0 | 20.7 | 21.9 | 24.7 | 16.1 | 16.4 |
| Drinking water Huizen | N/A | N/A | N/A | N/A | N/A | N/A | N/A | N/A | N/A | N/A |
| Drinking water Vianen | N/A | N/A | N/A | N/A | N/A | N/A | N/A | N/A | 24.3 | N/A |
| Drinking water Nieuwegein | N/A | N/A | N/A | N/A | N/A | N/A | N/A | N/A | N/A | N/A |
| Blank recycling | N/A | N/A | N/A | N/A | N/A | N/A | N/A | N/A | N/A | N/A |

Table 5 gives the results of determination of ten viruses before and after a reverse osmosis (RO) membrane. The results are the output of Q-PCR and are presented as Ct values. The virus DNA is multiplied during the PCR reaction. After a number of PCR cycles, sufficient DNA is present to exceed a set limit value. This is stated as the Ct value (number of cycles required to exceed the limit value). When many of the above viruses are present in the water (for example virus 2310), then a large amount of DNA of that virus is also present. Therefore fewer PCR cycles are needed to reach the limit value. Thus, for example virus 2019 is present in a low concentration, and therefore more PCR cycles are needed to reach the limit value.

The 1st row shows that the viruses present are in the surface water of the location, and the 2nd row shows that, apart from virus 2019, the viruses are no longer present in the water after the RO membrane.

The 3rd and 4th rows show the conversion of Ct value to log removal. Viruses most prevalent in the water (2310 and 2314) show a log removal of more than 6 log. The other rows show that the viruses mainly occur in surface water, but hardly in other sources.

Although the example described here shows a purification process of the reverse osmosis (RO) membrane type, it will be clear to a person skilled in the art that other types of purification processes are also suitable, as mentioned in the introduction of the present description.

## Claims

1. Method for determining the effectiveness of removal of viruses in a purification process for producing an aqueous stream, wherein the viruses present in a water sample are used for investigating the effectiveness of the purification process, said method comprising the following steps:
i) taking a water sample, at a point located upstream of the purification process;
ii) taking a water sample, at a point located downstream of the purification process,
wherein the water sample is taken from a water source, selected from the group of salt water, brackish water, groundwater, surface water, seawater, sewage treatment plant effluent, storm water, rainwater, and process water, or a combination thereof;
iii) isolating genetic material from one or more viruses present in the aforementioned water samples, obtained according to steps i) and ii), wherein the concentration of said one or more viruses in said water sample i) is in a range from more than one million to 10 billion per litre of water;
iv) carrying out a technique for multiplying RNA and/or DNA on the genetic material obtained according to step iii);
v) based on the results obtained from step iv), establishing the number of gene copies of specific virus types in the water sample obtained according to step i);
vi) based on the results obtained from step iv), establishing the number of gene copies of specific virus types in the water sample obtained according to step ii);
vii) determining the effectiveness of removal of viruses in the aforementioned purification process based on comparing the values obtained in step v) and vi), wherein in step iii) the genetic material is DNA and/or RNA, and when genetic material of the RNA type is present in step iii), step iv) is preceded by a step of synthesis of cDNA from the RNA,
wherein there is no dosage of markers in the water samples to be taken in step i) and/or step ii).

2. Method according to claim 1, **characterized in that** in step iv), one or more techniques are applied as the technique for multiplying RNA and/or DNA, selected from the group of polymerase chain reaction (PCR), in particular of the qPCR type, nucleic acid sequence based amplification (NASBA), loop mediated isothermal amplification (LAMP), self-sustained sequence replication (3SR) and rolling circle amplification (RCA).

3. Method according to one or more of the preceding claims, **characterized in that** the aforementioned one or more viruses of step iii) are determined beforehand.

4. Method according to Claim 3, **characterized in that** the aforementioned one or more viruses of step iii) are determined beforehand on the basis of one or more aspects, selected from the group of size of the aforementioned one or more viruses, hydrophobicity of the aforementioned one or more viruses and isoelectric point of the aforementioned one or more viruses.

5. Method according to one or more of the preceding claims, **characterized in that** the aforementioned purification process is selected from the group of coagulation, flocculation, sedimentation, flotation, lamella separation, sand filtration, such as rapid filtration and (biological) slow sand filtration, membrane distillation, (biological) activated-carbon filtration, ion exchange, bank filtration and passage through soil, passage through sand dunes, pressure-driven membrane processes, such as microfiltration (MF), ultrafiltration (UF), nanofiltration (NF), reverse osmosis (RO), forward osmosis (FO), electrodialysis (ED), oxidative processes (which are also partly to be regarded as disinfection processes), such as AOP UV/H₂O₂/O₃, UV, Cl₂, ClO₂, O₃, UV/O₃, Fenton, and UV-peroxide treatment, ARP (Advanced reduction processes), in particular using UV and sulphite, adsorptive processes (AKF, IEX), biological processes (BAKF, BAZF, dune, bank, soil), dosing of water treatment chemicals, such as acids, antiscalants, biocides, coagulants (Fe, Al), flocculants, or a combination thereof.

6. Method according to Claim 5, **characterized in that** the aforementioned purification process comprises nanofiltration and/or reverse osmosis (RO).

7. Method according to one or more of the preceding claims, **characterized in that** the aforementioned aqueous stream is selected from the group of irrigation water, process water, demineralized water, boiler feed water and drinking water, or a combination thereof.

8. Method according to one or more of the preceding claims, **characterized in that** the ratio of the volume of the water sample to be taken in step i) to the volume of the water sample to be taken in step ii) is in the range 1:1-1:10, preferably 1:1-1:5, wherein the magnitude of the volume of the water sample to be taken in step i) is in the range from 0.1 l to 1.5 l.

9. Method according to one or more of the preceding claims, **characterized in that** an LRV (log removal value) of at least 4 log units, preferably at least 6, in particular at least 7, is obtained.

## Patentansprüche

1. Verfahren zum Bestimmen der Wirksamkeit der Virenentfernung bei einem Aufreinigungsprozess zum Herstellen eines wässrigen Stroms, wobei die in einer Wasserprobe vorhandenen Viren zum Untersuchen der Wirksamkeit des Aufreinigungsprozesses verwendet werden, wobei das Verfahren die folgenden Schritte umfasst:
i) Entnehmen einer Wasserprobe an einem Punkt, der dem Aufreinigungsprozess vorgeschaltet ist;
ii) Entnehmen einer Wasserprobe an einem Punkt, der dem Aufreinigungsprozess nachgeschaltet ist,
wobei die Wasserprobe einer Wasserquelle entnommen wird, die aus der Gruppe aus Salzwasser, Brackwasser, Grundwasser, Oberflächenwasser, Meerwasser, Abwasserkläranlagenabfluss, Sturmwasser, Regenwasser und Prozesswaser oder einer Kombination davon ausgewählt ist;
iii) Isolieren von genetischem Material eines oder mehrerer Viren, die in den zuvor genannten Wasserproben, die gemäß Schritt i) und ii) erhalten wurden, vorhanden sind, wobei die Konzentration der einen oder mehreren Viren in der Wasserprobe i) in einem Bereich von mehr als einer Millionen bis zu 10 Milliarden pro Liter Wasser liegt;
iv) Durchführen einer Technik zum Multiplizieren von RNA und/oder DNA mit dem genetischen Material, das gemäß Schritt iii) erhalten wurde;
v) Ermitteln der Anzahl von Genkopien spezifischer Virustypen in der gemäß Schritt i) erhaltenen Wasserprobe basierend auf den aus Schritt iv) erhaltenen Ergebnissen;
vi) Ermitteln der Anzahl an Genkopien spezifischer Virustypen in der gemäß Schritt ii) erhaltenen Wasserprobe basierend auf den aus Schritt iv) erhaltenen Ergebnissen;
vii) Bestimmen der Wirksamkeit der Virenentfernung bei dem zuvor genannten Aufreinigungsprozess basierend auf dem Vergleichen der in Schritt v) und vi) erhaltenen Werte, wobei es sich bei dem genetischen Material in Schritt iii) um DNA und/oder RNA handelt und, wenn genetisches Material des RNA-Typs in Schritt iii) vorhanden ist, Schritt iv) ein Schritt der cDNA-Synthese aus der RNA vorgestellt ist,
wobei keine Markerdosierung in den Wasserproben, die in Schritt i) und/oder Schritt ii) entnommen werden sollen, vorhanden ist.

2. Verfahren nach Anspruch 1, **dadurch gekennzeichnet, dass** in Schritt iv) eine oder mehrere Techniken als Technik zum Multiplizieren von RNA und/oder DNA angewendet werden, die aus der Gruppe Polymerase Kettenreaktion (PCR= polymerase chain reaction), insbesondere qPCR, sequenzbasierte Amplifikation von Nukleinsäure (NASBA= nucleic acid sequence based amplification), schleifenvermittelte isotherme Amplifikation (LAMP= loop mediated isothermal amplification), selbststützende Sequenzreplikation (3SR= self-sustained sequence replication) und Rolling-Circle-Amplifikation (RCA) ausgewählt sind.

3. Verfahren nach einem oder mehreren der vorhergehenden Ansprüche, **dadurch gekennzeichnet, dass** die zuvor genannten ein oder mehreren Viren aus Schritt iii) zuvor bestimmt werden.

4. Verfahren nach Anspruch 3, **dadurch gekennzeichnet, dass** die zuvor genannten ein oder mehreren Viren aus Schritt iii) zuvor auf der Basis einer oder mehrerer Aspekte, die aus der Gruppe Größe der zuvor genannten ein oder mehreren Viren, Hydrophobie der zuvor genannten ein oder mehreren Viren und isoelektrischer Punkt der zuvor genannten ein oder mehreren Viren ausgewählt sind, bestimmt werden.

5. Verfahren nach einem oder mehreren der vorhergehenden Ansprüche, **dadurch gekennzeichnet, dass** der zuvor genannte Aufreinigungsprozess aus der Gruppe Koagulation, Flockung, Sedimentierung, Flotation, Lamellenseparation, Sandfiltrierung, wie rapide Filtrierung und (biologische) langsame Sandfiltrierung, Membrandestilliation, (biologische) Aktivkohlefiltrierung, Ionenaustausch, Bankfiltrierung und Versickern durch Erdboden, Versickern durch Sanddünen, druckgetriebene Membranprozesse wie zum Beispiel Mikrofiltrierung (MF), Ultrafiltrierung (UF), Nanofiltrierung (NF), Umkehrosmose (RO= reverse osmosis), Vorwärtsosmose (FO= forward osmosis), Elektrodialyse (ED), oxidative Prozesse (die auch teilweise als Desinfektionsprozesse angesehen werden), wie etwa AOP UV/H₂O₂/O₃-, UV-, Cl₂-, ClO₂-, O₃-, UV/O₃-, Fenton- und UV-Peroxid-Behandlung, ARP (Advanced reduction processes= fortgeschrittene Reduktionsverfahren), insbesondere unter Verwendung von UV und Sulfit, Adsorptionsverfahren (AKF, IEX), biologische Prozesse (BAKF, BAZF, Düne, Bank, Erdboden), Dosieren von Chemikalien zur Wasserbehandlung, wie etwa Säuren, Antiscalantien, Biozide, Koagulantien (Fe, Al), Flockungsmittel oder eine Kombination davon ausgewählt ist.

6. Verfahren nach Anspruch 5, **dadurch gekennzeichnet, dass** der zuvor genannte Aufreinigungsprozess Nanofiltrierung und/oder Umkehrosmose (RO= reverse osmosis) umfasst.

7. Verfahren nach einem oder mehreren der vorhergehenden Ansprüche, **dadurch gekennzeichnet, dass** der zuvor genannte wässrige Strom aus der Gruppe Irrigationswasser, Prozesswasser, demineralisiertem Wasser, Speisewasser und Trinkwasser oder einer Kombination davon ausgewählt ist.

8. Verfahren nach einem oder mehreren der vorhergehenden Ansprüche, **dadurch gekennzeichnet, dass** sich das Verhältnis von dem Volumen der Wasserprobe, die in Schritt i) entnommen werden soll, zu dem Volumen der Wasserprobe, die in Schritt ii) entnommen werden soll, im Bereich von 1:1-1:10, vorzugsweise 1:1-1:5 befindet, wobei sich die Größe des Volumens der Wasserprobe, die in Schritt i) entnommen werden soll, im Bereich von 0,1 l bis 1,5 l befindet.

9. Verfahren nach einem oder mehrerer der vorhergehenden Ansprüche **dadurch gekennzeichnet, dass** ein LRV (log removal value= log-Reduktionswert) von mindestens 4 log-Einheiten, vorzugsweise 6, insbesondere mindestens 7, erhalten wird.

## Revendications

1. Procédé de détermination de l'efficacité de l'élimination de virus dans un procédé de purification pour produire un flux aqueux, dans lequel les virus présents dans un échantillon d'eau sont utilisés pour étudier l'efficacité du procédé de purification, ledit procédé comprenant les étapes suivantes :
i) de prélèvement d'un échantillon d'eau, à un point situé en amont du procédé de purification ;
ii) de prélèvement d'un échantillon d'eau, à un point situé en aval du procédé de purification,
dans lequel l'échantillon d'eau est prélevé à partir d'une source d'eau, choisie dans le groupe d'eau salée, d'eau saumâtre, d'eau souterraine, d'eau de surface, d'eau de mer, d'effluent de station d'épuration des eaux d'égout, d'eau pluviale, d'eau de pluie et d'eau de traitement, ou d'une combinaison de ceux-ci ;
iii) d'isolement de matériel génétique d'un ou de plusieurs virus présents dans les échantillons d'eau susmentionnés, obtenus selon les étapes i) et ii), où la concentration dudit ou desdits plusieurs virus dans ledit échantillon d'eau i) se trouve dans une plage allant de plus d'un million à 10 milliards par litre d'eau ;
iv) de réalisation d'une technique de multiplication d'ARN et/ou d'ADN sur le matériel génétique obtenu selon l'étape iii) ;
v) sur la base des résultats obtenus à l'étape iv), d'établissement du nombre de copies de gènes de types de virus spécifiques dans l'échantillon d'eau obtenu selon l'étape i) ;
vi) sur la base des résultats obtenus à l'étape iv), d'établissement du nombre de copies de gènes de types de virus spécifiques dans l'échantillon d'eau obtenu selon l'étape ii) ;
vii) de détermination de l'efficacité de l'élimination de virus dans le procédé de purification susmentionné sur la base de la comparaison des valeurs obtenues aux étapes v) et vi), où à l'étape iii) le matériel génétique est de l'ADN et/ou de l'ARN, et lorsque le matériel génétique de type ARN est présent à l'étape iii), l'étape iv) est précédée d'une étape de synthèse d'ADNc à partir de l'ARN,
dans lequel il n'y a pas de dosage de marqueurs dans les échantillons d'eau à prélever à l'étape i) et/ou à l'étape ii).

2. Procédé selon la revendication 1, **caractérisé en ce qu'**à l'étape iv), une ou plusieurs techniques sont appliquées comme étant la technique de multiplication d'ARN et/ou d'ADN, choisie dans le groupe de la réaction en chaîne par polymérase (PCR), en particulier de type qPCR, de l'amplification basée sur une séquence d'acide nucléique (NASBA), de l'amplification isotherme à médiation par boucle (LAMP), de la réplication de séquences auto-entretenues (3SR) et de l'amplification en cercle roulant (RCA).

3. Procédé selon une ou plusieurs des revendications précédentes, **caractérisé en ce que** le ou les plusieurs virus susmentionnés de l'étape iii) sont déterminés au préalable.

4. Procédé selon la revendication 3, **caractérisé en ce que** le ou les plusieurs virus susmentionnés de l'étape iii) sont déterminés au préalable sur la base d'un ou de plusieurs aspects, choisis dans le groupe de la taille du ou des plusieurs virus susmentionnés, de l'hydrophobicité du ou des plusieurs virus susmentionnés et du point isoélectrique du ou des plusieurs virus susmentionnés.

5. Procédé selon une ou plusieurs des revendications précédentes, **caractérisé en ce que** le procédé de purification susmentionné est choisi dans le groupe de la coagulation, la floculation, la sédimentation, la flottation, la séparation par lamelles, la filtration sur sable, telle que la filtration rapide et la filtration lente sur sable (biologique), la distillation membranaire, la filtration sur charbon actif (biologique), l'échange d'ions, la filtration sur berge et le passage à travers le sol, le passage à travers les dunes de sable, les procédés à membrane sous pression, tels que la microfiltration (MF), l'ultrafiltration (UF), la nanofiltration (NF), l'osmose inverse (RO), l'osmose directe (FO), l'électrodialyse (ED), les procédés oxydatifs (qui doivent également être considérés en partie comme des procédés de désinfection), tels que AOP UV/H₂O₂/O₃, UV, Cl₂, ClO₂, O₃, UV/O₃, Fenton et le traitement au peroxyde-UV, ARP (procédés de réduction avancés), en particulier en utilisant l'UV et le sulfite, les procédés d'adsorption (AKF, IEX), les processus biologiques (BAKF, BAZF, dune, berge, sol), le dosage de produits chimiques de traitement de l'eau, tels que des acides, des antitartres, des biocides, des coagulants (Fe, Al), des floculants ou une combinaison de ceux-ci.

6. Procédé selon la revendication 5, **caractérisé en ce que** le procédé de purification susmentionné comprend la nanofiltration et/ou l'osmose inverse (RO).

7. Procédé selon une ou plusieurs des revendications précédentes, **caractérisé en ce que** le flux aqueux susmentionné est choisi dans le groupe de l'eau d'irrigation, de l'eau de traitement, de l'eau déminéralisée, de l'eau d'alimentation de chaudière et de l'eau potable, ou d'une combinaison de celles-ci.

8. Procédé selon une ou plusieurs des revendications précédentes, **caractérisé en ce que** le rapport du volume de l'échantillon d'eau à prélever à l'étape i) sur le volume de l'échantillon d'eau à prélever à l'étape ii) se trouve dans la plage allant de 1 : 1 à 1 : 10, de préférence de 1 : 1 à 1 : 5, où la grandeur du volume de l'échantillon d'eau à prélever à l'étape i) se trouve dans la plage allant de 0,1 l à 1,5 l.

9. Procédé selon une ou plusieurs des revendications précédentes, **caractérisé en ce qu'**une LRV (valeur d'élimination logarithmique) d'au moins 4 unités logarithmiques, de préférence d'au moins 6, en particulier d'au moins 7, est obtenue.
